# EUROPÄISCHE PATENTSCHRIFT

(11) **EP 1 268 426 B1**
(45) Veröffentlichungstag und Bekanntmachung des Hinweises auf die Patenterteilung: **18.09.2013**
(21) Anmeldenummer: 01927819.1
(22) Anmeldetag: 28.03.2001
(51) Int. Cl.: C07D 211/58, C08K 5/34

(54) **4-FORMYLAMINO-N-METHYLPIPERIDINDERIVATE, DEREN VERWENDUNG ALS STABILISATOREN UND DAMIT STABILISIERTES ORGANISCHES MATERIAL**
4-FORMYL AMINO-N-METHYLPIPERIDINE DERIVATIVES, THE USE THEREOF AS STABILISERS AND ORGANIC MATERIAL STABILISED THEREWITH
DERIVES DE 4-FORMYLAMINO-N-METHYLPIPERIDINE, LEUR UTILISATION EN TANT QUE STABILISATEURS ET MATIERE ORGANIQUE STABILISEE AU MOYEN DE CEUX-CI

(30) Priorität: 04.04.2000 DE 10016379
(43) Veröffentlichungstag der Anmeldung: 02.01.2003
(73) Patentinhaber: BASF SE, 67056 Ludwigshafen (DE)
(72) Erfinder: HAREMZA, Sylke, 69151 Neckargemünd (DE); KROCKENBERGER, Jürgen, 70569 Stuttgart (DE); APPEL, Manfred, 76829 Landau (DE); TRAUTH, Hubert, 67373 Dudenhofen (DE); AUMÜLLER, Alexander, 67435 Neustadt (DE)
(86) Internationale Anmeldenummer: PCT/EP2001/003503
(87) Internationale Veröffentlichungsnummer: WO 2001/074777

(56) Entgegenhaltungen:
- EP-A- 0 316 582

## Beschreibung

Die vorliegende Erfindung betrifft 4-Formylamino-N-methylpiperidinderivate der allgemeinen Formel (I) 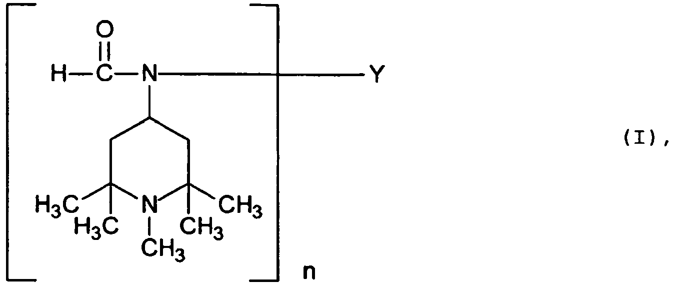 in welcher n und Y die in der Beschreibung angegebene Bedeutung besitzen, ein Verfahren zur Herstellung dieser Piperidinderivate sowie die Verwendung der erfindungsgemäßen oder der erfindungsgemäß hergestellten Piperidinderivate zum Stabilisieren von organischem Material, insbesondere von Kunststoffen und Lacken.

Weiter betrifft die Erfindung die Verwendung der erfindungsgemäßen oder der erfindungsgemäß hergestellten Piperidinderivate als Lichtschutzmittel und Stabilisatoren für Holzoberflächen.

Außerdem betrifft die Erfindung stabilisiertes organisches Material, welches erfindungsgemäße oder erfindungsgemäß hergestellte Piperidinderivate enthält.

2,2,6,6-Tetraalkylpiperidinderivate sind als Lichtschutzmittel für organische Polymere bekannt. Unbefriedigend ist oft die mangelhafte Verträglichkeit mit Polyolefinen und anderen Kunststoffen sowie die Unverträglichkeit mit Säuren oder mit Materialien, welche bei Einwirkung von Licht und/oder Wärme Säuren bilden, die Dauer der Schutzwirkung, die Eigenfarbe der Substanzen und die thermische Zersetzung der Stabilisatoren beim Einarbeiten in Polymere bei erhöhter Temperatur.

In der Offenlegungsschrift EP 0 316 582 werden Piperidinderivate mit diesbezüglich verbessertem Eigenschaftsprofil beschrieben, welche der Formel 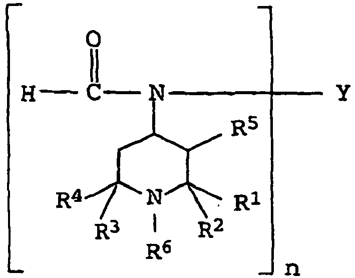 entsprechen. Hierin kommen R¹ bis R⁵, n und Y ähnliche Bedeutungen zu, wie in den Verbindungen der Formel I gemäß vorliegender Erfindung.

R⁶ umfasst, neben vielen anderen Resten, u.a. auch Wasserstoff und C₁-C₂₂-Alkyl. Auf Seite 4, Zeile 14 der Schrift EP 0 316 582 wird als bevorzugte Bedeutung von R⁶, außer Methyl, Acetyl, Cyanmethyl und Aminoethyl, insbesondere Wasserstoff aufgeführt. Dies kommt auch in den Verbindungen der Beispiele 1 bis 48 zum Ausdruck, in welchen R⁶ ausnahmslos Wasserstoff ist. Lediglich in der Verbindung des letzten Beispiels (Beispiel 49) ist das Stickstoffatom des Piperidinringes mit einem Acetylrest substituiert.

So gut das übrige Eigenschaftsprofil dieser Stabilisatoren auch ist, so lässt deren Stabilisierungsdauer oftmals jedoch noch zu wünschen übrig.

Der Erfindung lag daher die Aufgabe zugrunde, Stabilisatoren zur Verfügung zu stellen, welche eine weitere Verbesserung in der Dauer der Stabilisierung von organischem Material mit sich bringen, darüber hinaus aber die bereits erwähnten positiven Eigenschaften der bekannten Stabilisatoren, wie z.B. gute Verträglichkeit mit den zu stbilisierenden Materialien, etwa Polyolefinen und anderen Kunststoffen, geringe Eigenfarbe und Beständigkeit bei der Einarbeitung in das zu stabilisierende Material, aufweisen.

Überraschenderweise wurde diese Aufgabe mit Hilfe der 4-Formylamino-N-methylpiperidinderivate der allgemeinen Formel (I) 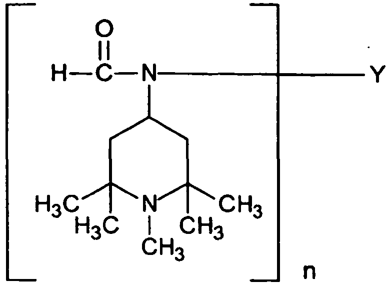 gelöst, in welcher bedeuten
n einen Wert von 1 oder 2, mit der Maßgabe, dass
   für n gleich 1:
Y Wasserstoff oder C₁-C₂₂-Alkyl, und
   für n gleich 2:
Y C₁-C₂₂-Alkylen bedeuten.

Die erfindungsgemäßen Verbindungen haben keine Eigenfarbe, sind gut verträglich mit verschiedensten organischen Materialien, insbesondere organischen Polymeren, zeigen einen niedrigen Dampfdruck und damit geringe Füchtigkeit, sind stabil gegen thermische Zersetzung und Säureeinwirkung und besitzen eine gegenüber den Verbindungen aus dem Stand der Technik verbesserte Stabilisierungsdauer.

Wenn n den Wert 1 annimmt, kommen für Y, neben Wasserstoff, in Betracht:
C₁-C₂₂-Alkyl, wie z.B. Methyl, Ethyl, n- und i-Propyl, n-, sec.- und i-Butyl, n- und i-Pentyl, Hexyl, Octyl, Decyl, Dodecyl, Octadecyl, Pivalyl, 3,3-Dimethylbutyl-2, Neopentyl, 4-Methylpentyl-2 und 2-Ethylhexyl;

Bevorzugte Piperidinderivate der Formel I sind weiter solche, in welchen n den Wert 2 annimmt und in welchen es sich bei Y um zweiwertige C₂-C₂₂-Alkylenbrücken handelt. Insbesonders kommen für Y C₁-C₂₂-Alkylenbrücken in Betracht.

Die efindungsgemäßen Verbindungen der allgemeinen Formel (I) können, wie auch in der Schrift EP 0 316 582 beschrieben, durch Reaktion von Verbindungen der allgemeinen Formel (II) 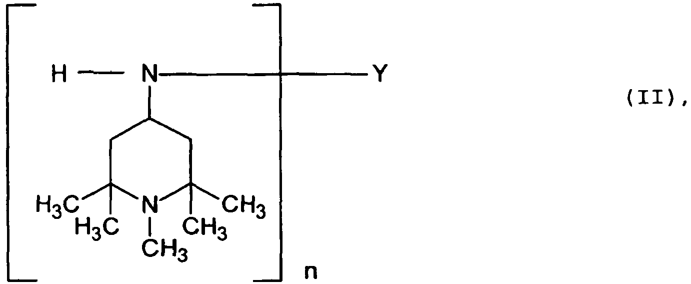 mit Ameisensäure oder Ameisensäureestem hergestellt werden, wobei bei letzteren der Methyl- oder Ethylester bevorzugt verwendet wird. Hierbei kann man ohne oder mit Zugabe eines Katalysators arbeiten. Als solche kommen vor allem Lewissäuren in Frage, von welchen insbesondere Titanorthoester, speziell Titanorthobutylat, erwähnt seien.

Ein weiterer Zugang zu den erfindungsgemäßen, methylierten Verbindungen erschließt sich durch Umsetzung der Piperidinderivate der Formel I' 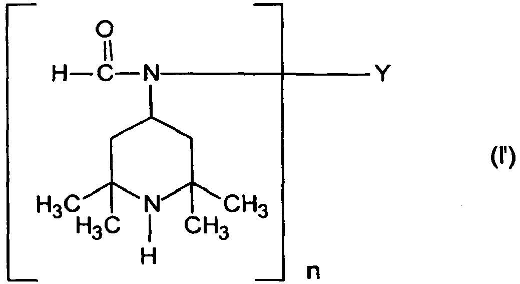 in welcher n und Y die zu Formel I angegebene Bedeutung besitzen, mit Methylierungsreagenzien, wie z.B. Methylhalogeniden, etwa Methylchlorid, -bromid oder-iodid, oder auch Dimethylsulfat. Diese Methylierungsreaktionen sind dem Fachmann vertraut und bedürfen daher keiner weiteren Erläuterungen.

Ein besonders elegantes und im Rahmen der vorliegenden Erfindung beanspruchtes Verfahren zur Herstellung von Piperidinderivaten der Formel I geht ebenfalls von den zuvor gezeigten Verbindungen der Formel I' aus, welche in einem organischen Lösungs-, Emulgier- oder Suspendiermittel (im Folgenden als "LESmittel" bezeichnet), dessen Siedepunkt (bei 1,013 bar) mindestens 70°C beträgt, mit Formaldehyd und anschließend bei einer Temperatur von 70°C bis maximal der Siedetemperatur (bei 1,013 bar) des verwendeten LESmittels mit Ameisensäure umgesetzt werden.

Diese Ausgangsamine sind zumeist kommerziell erhältlich, z.B. Uvinul^{®} 4050 H 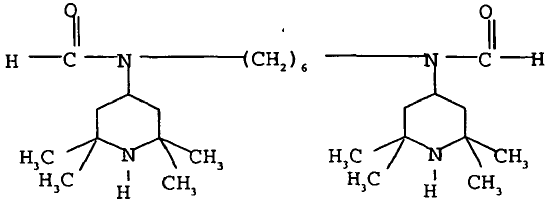 (BASF Aktiengesellschaft, Ludwigshafen). Gemäß Formel I entsprechen hierbei n dem Wert 2 und Y einer Hexamethylenbrücke.

Unter Formaldeyhd sei hierbei nicht nur die gasförmige Verbindung oder deren Lösung in Wasser (Formalin) verstanden, vielmehr können dies auch solche Vorläuferverbindungen sein, welche in leichter Weise Formaldehyd freisetzen. Beispielsweise sind dies die Halb- oder Vollacetale des Formaldehyds mit Alkanolen, wie z.B. Methanol oder Ethanol, mithin also die Verbindungen H₂C(OH)(OCH₃), H₂C(OCH₃)₂, H₂C(OH)(OC₂H₅) und H₂C(OC₂H₅)₂ und gegebenenfalls deren Mischungen.

Als organische LESmittel mit einem Siedepunkt (bei 1,013 bar) von mindestens 70°C eignen sich vor allem aromatische Kohlenwasserstoffe wie Toluol, Xylole und deren Gemische, Mesitylen und die Halogen- oder Nitrokohlenwasserstoffe, wie z.B. Chlorbenzol, die Dichlorbenzole oder Nitrobenzol. Insbesondere technische Gemische solcher Aromaten sind von Bedeutung. Daneben sind aber auch aromatenfreie Kohlenwasserstoffgemische einsetzbar, wenn sie einen entsprechend hohen Siedepunkt bzw. -bereich aufweisen.

Die Umsetzung der Verbindungen der Formel I' mit Formaldehyd im ersten Schritt erfolgt üblicherweise bei Temperaturen von 0 bis 80°C, vorzugsweise bei 20 bis 70°C, insbesondere bei Raumtemperatur.

Das molare Verhältnis von N-H-Gruppen, welche in N-CH₃-Gruppen überführt werden sollen, und Formaldehyd liegt im Bereich von 1:1,25 bis 1:2,5, d.h. bei einem molaren Formaldehyd-Überschuss von 25 bis 150%. Üblicherweise beträgt das molare Verhältnis etwa 1:2, d.h. Formaldehyd wird in 100 mol-%igem Überschuss gegenüber der für die stöchiometrische Umsetzung benötigten Menge verwendet.

Die Umsetzungstemperatur des Formaldehydadduktes mit Ameisensäure liegt üblicherweise bei Raumtemperatur bis zur Siedetemperatur (bei 1,013 bar) des verwendeten LESmittels, vorzugsweise bei einer Temperatur von 40°C bis maximal der Siedetemperatur (bei 1,013 bar) des verwendeten LESmittels.

Das bei der Umsetzung gebildete Wasser wird zweckmäßigerweise azeotrop abdestilliert, wobei man das LESmittel nach dessen Abtrennung vom Reaktionswasser wieder in das Reaktionsgemisch zurückführen kann.

Die Umsetzung wird üblicherweise bei Normaldruck (1,013 bar) durchgeführt, sie kann jedoch auch bei reduziertem Druck oder unter Durchleitung eines Inertgases, wie z.B. Stickstoff, erfolgen. Hierdurch wird die Entfernung des bei der Umsetzung entstehenden Kohlendioxids unterstützt.

Erwähnt sei hier, dass die erfindungsgemäßen sowie erfindungsgemäß hergestellten Verbindungen üblicherweise in Form der freien Basen Verwendung finden. Darüber hinaus können diese Verbindungen aber auch in Form ihrer Säureaddukte verwendet werden. Geeignete Anionen leiten sich in diesem Fall z.B. von anorganischen Säuren und insbesondere von organischen Carbonsäuren sowie organischen Sulfonsäuren ab.

Als anorganische Anionen sind z.B. Chlorid, Bromid, Sulfat, Methosulfat, Tetrafluoroborat, Phosphat und Rhodanid zu nennen.

Als Carbonsäureanionen kommen z.B. Formiat, Acetat, Propionat, Hexanoat, Cyclohexanoat, Lactat, Polycarbonsäuren mit bis zu 3000 COOH-Gruppen in Betracht.

Sulfonsäure-Anionen sind beispielsweise Benzolsulfonat oder Tosylat.

Erfindungsgemäß verwendet man die Piperidinderivate der Formel I sowie die nach dem erfindungsgemäßem Verfahren hergestellten Piperidinderivate zum Stabilisieren von organischem Material,insbesondere gegen den Abbau durch Licht und Wärme. Darüber hinaus sei hier auch deren Wirksamkeit als Metalldesaktivatoren erwähnt.

Unter organischem Material sind insbesondere Kunststoffe (synonym wird hier auch der Begriff Polymere gebraucht) und Lacke, aber auch Holzoberflächen jeglicher Art zu verstehen.

Die Piperidinderivate werden dem zu stabilisierenden organischen Material, insbesondere den zu stabilisierenden Kunststoffen, vorzugsweise in einer Konzentration von 0,01 bis 5 Gew.-%, insbesondere von 0,02 bis 1 Gew.-%, bezogen auf das organische Material zugesetzt. Wird das organische Material aus kleineren Molekülen aufgebaut, wie etwa bei der Herstellung von Kunststoffen aus den entsprechenden Monomeren, so kann die Zugabe vor, während oder nach der Synthese des organischen Materials geschehen.

Sollen Holzoberflächen geschützt werden, so sind die zuvor genannten Konzentrationen an erfindungsgemäßen und erfindungsgemäß hergestellten Verbindungen als gewichtsmäßige Anteile in entsprechenden Holzschutzformulierungen zu verstehen.

Zur Vermischung der erfindungsgemäßen und erfindungsgemäß hergestellten Verbindungen mit dem organischen Material, insbesondere den Kunststoffen und Lacken, können alle bekannten Vorrichtungen und Methoden zum Einmischen von Stabilisierungsmitteln oder anderen Zusätzen in organische Materialien, z.B. Polymere, angewandt werden.

Das durch die erfindungsgemäßen und erfindungsgemäß hergestellten Verbindungen stabilisierte organische Material, insbesondere Kunststoffe und Lacke, kann gegebenenfalls noch weitere Additive enthalten, z.B. Antioxidantien, Costabilisatoren, Lichtstabilisierungsmittel, Metalldesaktivatoren, antistatische Mittel, flammhemmende Mittel, Pigmente und Füllstoffe.

Antioxidanten, die den Kunststoffen neben den erfindungsgemäßen und erfindungsgemäß hergestellten Verbindungen zugesetzt werden können, sind z.B. Verbindungen, welche den Gruppen der
a) alkylierten Monophenole,
b) Alkylthiomethylphenole,
c) Hydrochinone und alkylierten Hydrochinone,
d) Tocopherole,
e) hydroxylierten Diphenylthioether,
f) Alkylidenbisphenole,
g) O-, N- und S-Benzylverbindungen,
h) aromatischen Hydroxybenzylverbindungen,
i) Triazinverbindungen,
j) Benzylphosphonate,
k) Acylaminophenole,
l) Ester der β-(3,5-Di-tert.-butyl-4-hydroxyphenyl)-propionsäure, β-(5-Tert.-butyl-4-hydroxy-3-methylphenyl)-propionsäure, β-(3,5-Dicyclohexyl-4-hydroxyphenyl)-propionsäure und 3,5-Di-tert.-butyl-4-hydroxyphenyl-essigsäure,
m) Amide der β-(3,5-Di-tert.-butyl-4-hydroxyphenyl)-propionsäure,
n) Ascorbinsäure und ihrer Derivate,
o) Antioxidantien auf Basis von Aminverbindungen,
p) Phosphite und Phosphonite,
q) 2-(2'-Hydroxyphenyl)-benzotriazole,
r) schwefelhaltigen Peroxidfänger bzw. schwefelhaltigen Antioxidantien
s) 2-Hydroxybenzophenone,
t) Ester der unsubstituierten und substituierten Benzoesäure,
u) Acrylate,
v) sterisch gehinderten Amine,
w) Oxamide und
x) 2-(2-Hydroxyphenyl)-1,3,5-triazine
zuzuordnen sind.

Zur Gruppe a) der alkylierten Monophenole zählen beispielsweise 2,6-Di-tert-butyl-4-methylphenol, 2-Tert-butyl-4,6-dimethylphenol, 2,6-Di-tert-butyl-4-ethylphenol, 2,6-Di-tert-butyl-4-n-butylphenol, 2,6-Di-tert-butyl-4-isobutylphenol, 2,6-Dicyclopentyl-4-methylphenol, 2-(α-Methylcyclohexyl)-4,6-dimethylphenol, 2,6-Dioctadecyl-4-methylphenol, 2,4,6-Tricyclohexylphenol, 2,6-Di-tert-butyl-4-methoxymethylphenol, Nonylphenole, welche eine lineare oder verzweigte Seitenkette besitzen, beispielsweise 2,6-Di-nonyl-4-methylphenol, 2,4-Dimethyl-6-(1'-methylundec-1'-yl)-phenol, 2,4-Dimethyl-6-(1'-methylheptadec-1'-yl)phenol, 2,4-Dimethyl-6-(1'-methyltridec-1'-yl)phenol und Mischungen dieser Verbindungen. Zur Gruppe b) der Alkylthiomethylphenole zählen beispielsweise 2,4-Dioctylthiomethyl-6-tert-butylphenol, 2,4-Dioctylthiomethyl-6-methylphenol, 2,4-Dioctylthiomethyl-6-ethylphenol und 2,6-Di-dodecylthiomethyl-4-nonylphenol.

Zur Gruppe c) der Hydrochinone und alkylierten Hydrochinone zählen beispielsweise 2,6-Di-tert-butyl-4-methoxyphenol, 2,5-Di-tert-butylhydrochinon, 2,5-Di-tert-amylhydrochinon, 2,6-Diphenyl-4-octadecyloxyphenol, 2,6-Di-tert-butylhydrochinon, 2,5-Di-tert-butyl-4-hydroxyanisol, 3,5-Di-tert-butyl-4-hydroxyanisol, 3,5-Di-tert-butyl-4-hydroxyphenylstearat und Bis-(3,5-di-tert-butyl-4-hydroxyphenyl)adipat.

Zur Gruppe d) der Tocopherole zählen beispielsweise α-Tocopherol, β-Tocopherol, γ-Tocopherol, δ-Tocopherol und Mischungen dieser Verbindungen, sowie Tocopherolderivate, wie beispielsweise Tocopherylacetat, -succinat, -nicotinat und -polyoxyethylensuccinat ("Tocofersolan").

Zur Gruppe e) der hydroxylierten Diphenylthioether zählen beispielsweise 2,2'-Thiobis(6-tert-butyl-4-methylphenol), 2,2'-Thiobis(4-octylphenol), 4,4'-Thiobis(6-tert-butyl-3-methylphenol), 4,4'-Thiobis(6-tert-butyl-2-methylphenol), 4,4'-Thiobis-(3,6-di-sec-amylphenol) und 4,4'-Bis-(2,6-dimethyl-4-hydroxyphenyl)disulfid.

Zur Gruppe f) der Alkylidenbisphenole zählen beispielsweise 2,2'-Methylenbis(6-tert-butyl-4-methylphenol), 2,2'-Methylenbis-(6-tert-butyl-4-ethylphenol), 2,2'-Methylenbis[4-methyl-6-(α-methylcyclohexyl)phenol], 2,2'-Methylenbis(4-methyl-6-cyclohexylphenol), 2,2'-Methylenbis(6-nonyl-4-methylphenol), 2,2'-Methylenbis(4,6-di-tert-butylphenol), 2,2-Ethylidenbis(4,6-di-tertbutylphenol), 2,2'Ethylidenbis(6-tert-butyl-4-isobutylphenol), 2,2'-Methylenbis[6-(α-methylbenzyl)-4-nonylphenol], 2,2'-Methylenbis[6-(α,α-dimethylbenzyl)-4-nonylphenol], 4,4'-Methylenbis(2,6-di-tert-butylphenol), 4,4'-Methylenbis-(6-tert-butyl-2-methylphenol), 1,1-Bis(5-tert-butyl-4-hydroxy-2-methylphenyl)butan, 2,6-Bis(3-tert-butyl-5-methyl-2-hydroxybenzyl)-4-4-methylphenol, 1,1,3-Tris(5-tert-butyl-4-hydroxy-2-methylphenyl)butan, 1,1-Bis(5-tert-butyl-hydroxy-2-methyl-phenyl)-3-n-dodecylmercaptobutan, Ethylenglycol-bis[3,3-bis(3'-tert-butyl-4'-hydroxyphenyl)butyrat], Bis(3-tert-butyl-4-hydroxy-5-methyl-phenyl)dicyclopentadien, Bis[2-(3'-tert-butyl-2'-hydroxy-5'-methylbenzyl)-6-tert-butyl-4-methylphenyl]-terephthalat, 1,1-Bis-(3,5-dimethyl-2-hydroxyphenyl)butan, 2,2-Bis-(3,5-ditert-butyl-4-hydroxyphenyl)propan, 2,2-Bis-(5-tert-butyl-4-hydroxy-2-methylphenyl)-4-n-dodecylmercaptobutan und 1,1,5,5-Tetrakis-(5-tert-butyl-4-hydroxy-2-methylphenyl)pentan.

Zur Gruppe g) der O-, N- und S-Benzylverbindungen zählen beispielsweise 3,5,3',5'-Tetra-tert-butyl-4,4'-dihydroxydibenzylether, Octadecyl-4-hydroxy-3,5-dimethylbenzylmercaptoacetat, Tridecyl-4-hydroxy-3,5-di-tert-butylbenzylmercaptoacetat, Tris(3,5-di-tert-butyl-4-hydroxybenzyl)-amin, Bis(4-tert-butyl-3-hydroxy-2,6-dimethylbenzyl)dithioterephthalat, Bis(3,5-di-tert-butyl-4-hydroxybenzyl)sulfid und Isooctyl-3,5-di-tert-butyl-4-hydroxybenzylmercaptoacetat.

Zur Gruppe h) der aromatischen Hydroxybenzylverbindungen zählen beispielsweise 1,3,5-Tris-(3,5-di-tert-butyl-4-hydroxybenzyl)-2,4,6-trimethylbenzol, 1,4-Bis(3,5-di-tert-butyl-4-hydroxybenzyl)-2,3,5,6-tetramethylbenzol und 2,4,6-Tris(3,5-di-tert-butyl-4-hydroxybenzyl)phenol.

Zur Gruppe i) der Triazinverbindungen zählen beispielsweise 2,4-Bis(octylmercapto)-6-(3,5-ditert-butyl-4-hydroxyanilino)-1,3,5-triazin, 2-Octylmercapto-4,6-bis(3,5-di-tert-butyl-4-hydroxyanilino)-1,3,5-triazin, 2-Octylmercapto-4,6-bis(3,5-di-tert-butyl-4-hydroxyphenoxy)-1,3,5-triazin, 2,4,6-Tris(3,5-di-tert-butyl-4-hydroxyphenoxy)-1,2,3-triazin, 1,3,5-Tris(3,5-ditert-butyl-4-hydroxybenzyl)isocyanurat, 1,3,5-Tris(4-tert-butyl-3-hydroxy-2,6-dimethylbenzyl)isocyanurat, 2,4,6-Tris(3,5-di-tert-butyl-4-hydroxyphenylethyl)-1,3,5-triazin, 1,3,5-Tris-(3,5-di-tert-butyl-4-hydroxyphenylpropionyl)-hexahydro-1,3,5-triazin, 1,3,5-Tris(3,5-dicyclohexyl-4-hydroxybenzyl)isocyanurat und 1,3,5-Tris(2-hydroxyethyl)isocyanurat.

Zur Gruppe j) der Benzylphosphonate zählen beispielsweise Dimethyl-2,5-di-tert-butyl-4-hydroxybenzylphosphonat, Diethyl-3,5-di-tert-butyl-4-hydroxybenzylphosphonat, Dioctadecyl-3,5-di-tert-butyl-4-hydroxybenzylphosphonat und Dioctadecyl-5-tert-butyl-4-hydroxy-3-methylbenzylphosphonat.

Zur Gruppe k) der Acylaminophenole zählen beispielsweise 4-Hydroxylauroylanilid, 4-Hydroxystearoylanilid und Octy)-N-(3,5-di-tert-butyl-4-hydroxyphenyl)carbamat.

Den Estern der in Gruppe I) genannten Propion- und Essigsäurederivate liegen ein- oder mehrwertige Alkohole zugrunde wie beispielsweise Methanol, Ethanol, n-Octanol, i-Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris(hydroxyethyl)isocyanurat, N,N'-Bis(hydroxyethyl)oxamid, 3-Thiaundecanol, 3-Thiapentadecanol, Trimethylhexandiol, Trimethylolpropan und 4-Hydroxymethyl-1-phospha-2,6,7-trioxabicyclo[2.2.2]-octan.

Den Amiden des in Gruppe m) genannten Propionsäurederivats liegen Aminderivate zugrunde wie beispielsweise N,N'-Bis(3,5-di-tert-butyl-4-hydroxyphenylpropionyl)hexamethylendiamin, N,N'-Bis(3,5-di-tert-butyl-4-hydroxyphenylpropionyl)trimethylendiamin und N,N'-Bis(3,5-di-tert-butyl-4-hydroxyphenylpropionyl)hydrazin.

Zur Gruppe n) zählen neben Ascorbinsäure (Vitamin C) auch Ascorbinsäurederivate wie beispielsweise Ascorbylpalmitat, -laurat und -stearat sowie Ascorbylsulfat und -phosphat.

Zur Gruppe o) der Antioxidantien auf Basis von Aminverbindungen zählen beispielsweise N,N'-Di-isopropyl-p-phenylendiamin, N,N'-Di-sec-butyl-p-phenylendiamin, N,N'-Bis(1,4-dimethylpentyl)-phenylendiamin, N,N'-Bis(1-ethyl-3-methylpentyl)-p-phenylendiamin, N,N'-Bis(1-methylheptyl)-p-phenylendiamin, N,N'-Dicyclohexyl-p-phenylendiamin, N,N'-Diphenyl-p-phenylendiamin, N,N'-Bis-(2-naphthyl)-p-phenylendiamin, N-Isopropyl-N'-phenyl-p-phenylendiamin, N-(1,3-Dimethylbutyl)-N'-phenyl-p-phenylendiamin, N-(1-Methylheptyl)-N'-phenyl-p-phenylendiamin, N-Cyclohexyl-N'-phenyl-p-phenylendiamin, 4-(p-Toluolsulfamoyl)-diphenylamin, N,N'-Dimethyl-N,N'-di-sec-butyl-p-phenylendiamin, Diphenylamin, N-Allyldiphenylamin, 4-Isopropoxydiphenylamin, N-Phenyl-1-naphthylamin, N-(4-Tert-octylphenyl)-1-naphthylamin, N-Phenyl-2-naphthylamin, octylsubstituiertes Diphenylamin, wie beispielsweise p,p'-Di-tert-octyldiphenylamin, 4-n-Butylaminophenol, 4-Butyrylaminophenol, 4-Nonanoylaminophenol, 4-Dodecanoylaminophenol, 4-Octadecanoylaminophenol, Bis[4-methoxyphenyl)amin, 2,6-Di-tert-butyl-4-dimethylaminomethylphenol, 2,4-Diaminodiphenylmethan, 4,4'-Diaminodiphenylmethan, N,N,N',N'-Tetramethyl-4,4'-di-aminidiphenylmethan, 1,2-Bis[(2-methylphenyl)amino]ethan, 1,2-Bis(phenylamino)propan, (o-Tolyl)biguanid, Bis[4-(1',3'-dimethylbutyl)phenyl]amin, tert-octyl substituiertes N-Phenyl-1-naphthylamin, eine Mischung von mono- und dialkyliertem Tert-butyl/Tert-octyldiphenylamin, eine Mischung von mono- und dialkyliertem Nonyldiphenylamin, eine Mischung von mono- und dialkyliertem Dodecyldiphenylamin, eine Mischung von mono-und dialkyliertem Isopropyl/Isohexyldiphenylamin, eine Mischung von mono- und dialkyliertem Tert-butyldiphenylamin, 2,3-Dihydro-3,3-dimethyl-4H-1,4-benzothiazin, Phenothiazin, eine Mischung von mono- und dialkyliertem Tert-butyl/Tert-octylphenothiazin, eine Mischung von mono- und dialkyliertem Tert-octylphenothiazin, N-Allylphenothiazin, N,N,N',N'-Tetraphenyl-1,4-diaminobut-2-en, N,N-Bis(2,2,6,6-tetramethyl-piperidin-4-yl)-hexamethylendiamin, Bis(2,2,6,6-tetramethylpiperidin-4-yl)sebacat, 2,2,6,6-Tetramethylpiperidin-4-on und 2,2,6,6-Tetramethylpiperidin-4-ol.

Zur Gruppe p) der Phosphite und Phosphonite zählen beispielsweise Triphenylphosphit, Diphenylalkylphosphit, Phenyldialkylphosphit, Tris(nonylphenyl)phosphit, Trilaurylphosphit, Trioctadecylphosphit, Distearylpentaerythritoldiphosphit, Tris(2,4-di-tert-butylphenyl)phosphit, Diisodecylpentaerythritoldiphosphit, Bis(2,4-di-tert-butylphenyl)pentaerythritoldiphosphit, Bis(2,6-ditert-butyl-4-methylphenyl)-pentaerythritoldiphosphit, Diisodecyloxypentaerythritoldiphosphit, Bis(2,4-di-tert-butyl-6-methylphenyl)-pentaerythritoldiphosphit, Bis(2,4,6-tris-(tert-butylphenyl))-pentaerythritoldiphosphit, Tristearylsorbitoltriphosphit, Tetrakis(2,4-di-tert-butylphenyl)-4,4'-biphenylendiphosphonit, 6-Isooctyloxy-2,4,8,10-tetra-tert-butyl-12H-dibenz-[d,g]-1,3,2-dioxaphosphocin, 6-Fluoro-2,4,8,10-tetra-tert-butyl-12-methyldibenz[d,g]-1,3,2-dioxaphosphocin, Bis(2,4-di-tert-butyl-6-methylphenyl)methylphosphit und Bis(2,4-di-tert-butyl-6-methylphenyl)-ethylphosphit.

Zur Gruppe q) der 2-(2'-Hydroxyphenyl)-benzotriazole zählen beispielsweise 2-(2'-Hydroxy-5'-methylphenyl)-benzotriazol, 2-(3',5'-Di-tert-butyl-2'-hydroxyphenyl)benzotriazol, 2-(5'-Tert--butyl-2'-hydroxyphenyl)benzotriazol, 2-(2'-Hydroxy-5'-(1,1,3,3-tetramethylbutyl)phenyl)benzotriazol, 2-(3',5'-Di-tert-butyl-2'-hydroxyphenyl)-5-chlorobenzotriazol, 2-(3'-Tert-butyl-2'-hydroxy-5'-methylphenyl)-5-chloro-benzotriazol, 2-(3'-Secbutyl-5'-tert-butyl-2'-hydroxyphenyl)-benzotriazol, 2-(2'-Hydroxy-4'-octyloxyphenyl)-benzotriazol, 2-(3',5'-Di-tert-amyl-2'-hydroxyphenyl)-benzotriazol, 2-(3',5'-Bis-(α,α-dimethylbenzyl)-2'-hydroxyphenyl)-benzotriazol, eine Mischung von 2-(3'-Tert-butyl-2'-hydroxy-5'-(2-octyloxycarbonylethyl)phenyl)-5-chloro-benzotriazol, 2-(3'-Tert-butyl-5'-[2-(2-ethylhexyloxy)-carbonylethyl]-2'-hydroxyphenyl)-5-chloro-benzotriazol, 2-(3'-Tert-butyl-2'-hydroxy-5'-(2-methoxycarbonylethyl)phenyl)-5-chloro-benzotriazol, 2-(3'-Tert-buty)-2-hydroxy-5'-(2-methoxycarbonylethyl)phenyl)-benzotriazol, 2-(3'-Tert-butyl-2'-hydroxy-5'-(2-octyloxycarbonylethyl)phenyl)benzotriazol, 2-(3'-Tert-butyl-5-[2-(2-ethylhexyloxy)carbonylethyl]-2'-hydroxyphenyl)-benzotriazol, 2-(3'-Dodecyl-2'-hydroxy-5'-methylphenyl)-benzotriazol und 2-(3'-Tert-butyl-2'-hydroxy-5'-(2-isooctyloxycarbonylethyl)-phenylbenzotriazol, 2,2-Methylen-bis[4-(1,1,3,3-tetramethylbutyl)-6-benzotriazol-2-ylphenol]; das Produkt der vollständigen Veresterung von 2-[3'-Tert-butyl-5'-(2-methoxycarbonylethyl)-2'-hydroxyphenyl]-2H-benzotriazol mit Polyethylenglycol 300; [R-CH₂CH₂-COO(CH₂)₃]₂, mit R = 3'-Tert-butyl-4'-hydroxy-5'-2H-benzotriazol-2-ylphenyl.

Zur Gruppe r) der schwefelhaltigen Peroxidfänger bzw. schwefelhaltigen Antioxidantien zählen z.B. Ester der 3,3'-Thiodipropionsäure, beispielsweise die Lauryl-, Stearyl-, Myristyl- oder Tridecylester, Mercaptobenzimidazol oder das Zinksalz des 2-Mercaptobenzimidazol, Zinkdibutyldithiocarbamat, Dioctadecyldisulfid und Pentaerythritoltetrakis(β-dodecylmercapto)propionat.

Zur Gruppe s) der 2-Hydroxybenzophenone zählen beispielsweise die 4-Hydroxy-, 4-Methoxy-, 4-Octyloxy-, 4-Decyloxy-, 4-Dodecyloxy-, 4-Benzyloxy-, 4.2',4-Trihydroxy- und 2'-Hydroxy-4,4'-dimethoxy-Derivate.

Zur Gruppe t) der Ester der unsubstituierten und substituierten Benzoesäure zählen beispielsweise 4-Tert-butylphenylsalicylat, Phenylsalicylat, Octylphenylsalicylat, Dibenzoylresorcinol, Bis(4-tert-butylbenzoyl)resorcinol, Benzoylresorcinol, 2,4-Di-tert-butylphenyl-3,5-di-tert-butyl-4-hydroxybenzoat, Hexadecyl-3,5-di-tert-butyl-4-hydroxybenzoat, Octadecyl-3,5-di-tert-butyl-4-hydroxybenzoat und 2-Methyl-4,6-di-tert-butylphenyl-3,5-di-tert-butyl-4-hydroxybenzoat.

Zur Gruppe u) der Acrylate zählen beispielsweise Ethyl-a-cyano-β,β-diphenylacrylat, Isooctyl-a-cyano-β,β-diphenylacrylat. Methyl-α-methoxycarbonylcinnamat, Methyl-α-cyano-β-methyl-p-methoxycinnamat, Butyl-α-cyano-β-methyl-p-methoxy-cinnamat und Methyl-α-methoxycarbonyl-p-methoxycinnamat.

Zur Gruppe v) der sterisch gehinderten Amine zählen beispielsweise Bis(2,2,6,6-tetramethylpiperidin-4-yl)sebacat, Bis(2,2,6,6-tetramethylpiperidin-4-yl)succinat, Bis(1,2,2,6,6-pentamethylpiperidin-4-yl)sebacat, Bis(1-octyloxy-2,2,6,6-tetramethylpiperidin-4-yl)sebacat, Bis(1,2,2,6,6-pentamethylpiperidin-4-yl)-n-butyl-3,5-di-tert-butyl-4-hydroxybenzylmalonat, das Kondensationsprodukt aus 1-(2-Hydroxyethyl)-2,2,6,6-tetramethyl-4-hydroxypiperidin und Bernsteinsäure, das Kondensationsprodukt aus N,N'-Bis(2,2,6,6-tetramethylpiperidin-4-yl)hexamethylendiamin und 4-Tert-octylamino-2,6-dichloro-1,3,5-triazin, Tris(2,2,6,6-tetramethylpiperidin-4-yl)nitrilotriacetat, Tetrakis(2,2,6,6-tetramethylpiperidin-4-yl)-1,2,3,4-butan-tetracarboxylat, 1,1'-(1,2-Ethylen)-bis(3,3,5,5-tetramethylpiperazinon), 4-Benzoyl-2,2,6,6-tetramethylpiperidin, 4-Stearyloxy-2,2,6,6-tetramethylpiperidin, Bis(1,2,2,6,6-pentamethylpiperidin-4-yl)-2-n-butyl-2-(2-hydroxy-3,5-di-tert-butylbenzyl)malonat, 3-n-Octyl-7,7,9,9-tetramethyl-1,3,8-triazaspiro[4.5]decan-2,4-dion, Bis(1-octyloxy-2,2,6,6-tetramethylpiperidin-4-yl)sebacat, Bis(1-octyloxy-2,2,6,6-tetramethylpiperidin-4-yl)succinat, das Kondensationsprodukt aus 2-Chloro-4,6-bis(4-n-butylamino-2,2,6,6-tetramethylpiperidin-4-yl)-1,3,5-triazin und 1,2-Bis(3--aminopropylamino)ethan, das Kondensationsprodukt aus 2-Chloro-4,6-di-(4-n-butylamino-1,2,2,6,6-pentamethylpiperidin-4-yl)-1,3,5-triazin und 1,2-Bis-(3-aminopropylamino)ethan, 8-Acetyl-3-dodecyl-7,7,9,9-tetramethyl-1,3,8-triazaspiro[4.5]-decan-2,4-dion, 3-Dodecyl-1-(2,2,6,6-tetramethylpiperidin-4-yl)pyrrolidin-2,5-dion, 3-Dodecyl-1-(1,2,2,6,6-pentamethylpiperidin-4-yl)pyrrolidin-2,5-dion, eine Mischung aus 4-Hexadecyloxy- und 4-Stearyloxy-2,2,6,6-tetramethylpiperidin, das Kondensationsprodukt aus N,N'-Bis-(2,2,6,6-tetramethylpiperidin-4-yl)-hexamethylendiamin und 4-Cyclohexylamino-2,6-dichloro-1,3,5-triazin, das Kondensationsprodukt aus 1,2-Bis(3-aminopropylamino)-ethan und 2,4,6-Trichloro-1,3,5-triazin, 4-Butylamino-2,2,6,6-tetramethylpiperidin, N-(2,2,6,6-Tetramethylpiperidin-4-yl)-n-dodecylsuccinimid, N-(1,2,2,6,6-Pentamethylpiperidin-4-yl)-n-dodecylsuccinimid, 2-Undecyl-7,7,9,9-tetramethyl-1-oxa-3,8-diaza-4-oxo-spiro[4.5]-decan, das Kondensationsprodukt aus 7,7,9,9-Tetramethyl-2-cycloundecyl-1-oxa-3,8-diaza-4-oxospiro-[4.5]decan und Epichlorhydrin, Poly(methoxypropyl-3-oxy)-[4-(2,2,6,6-tetramethyl)piperidinyl]siloxan, polymeranaloge Umsetzungsprodukte aus 4-Amino-2,2,6,6-tetramethylpiperidin und Maleinsäure/C₂₀-C₂₄-α-Olefin-Copolymeren, z.B. Uvinul^{®}5050 H (BASF Aktiengesellschaft, Ludwigshafen), und dazu entsprechende polymeranaloge Umsetzungsprodukte mit 4-Amino- 1,2,2,6,6-pentamethylpiperidin (z.B. "methyliertes Uvinul^{®}5050 H"), Kondensationsprodukte aus Tetramethylolacetylendihamstoff und 4-Amino-2,2,6,6-tetramethylpiperidin, z.B. Uvinul^{®}4049 H (BASF Aktiengesellschaft, Ludwigshafen), und dazu entsprechende Kondensationsprodukte mit 4-Amino-1,2,2,6,6-pentamethylpiperidin (z.B. "methyliertes Uvinul^{®}4049 H") sowie N,N'-Bis(2,2,6,6-tetramethylpiperidin-4-yl)-N,N'-bis-formyl-1,6-diaminohexan, z.B. Uvinul^{®}4050 H (BASF Aktiengesellschaft, Ludwigshafen).

Zur Gruppe w) der Oxamide zählen beispielsweise 4,4'-Dioctyloxyoxanilid, 2,2'-Diethoxyoxanilid, 2,2'-Dioctyloxy-5,5'-di-tert-butoxanilid, 2,2'-Didodecyloxy-5,5'-di-tert-butoxanilid, 2-Ethoxy-2'-ethyloxanilid, N,N'-Bis(3-dimethylaminopropyl)oxamid, 2-Ethoxy-5-tert-butyl-2'-ethoxanilid und dessen Mischung mit 2-Ethoxy-2'-ethyl-5,4'-di-tert-butoxanilld sowie Mischungen von ortho-, para-Methoxy-disubstituierten Oxaniliden und Mischungen von ortho- und para-Ethoxy disubstituierten Oxaniliden.

Zur Gruppe x) der 2-(2-Hydroxyphenyl)-1,3,5-triazine zählen beispielsweise 2,4,6-Tris(2-hydroxy-4-octyloxyphenyl)-1,3,5-triazin, 2-(2-Hydroxy-4-octyloxyphenyl)-4,6-bis(2,4-dimethylphenyl)-1,3,5-triazin, 2-(2,4-Dihydroxyphenyl)-4,6-bis(2,4-dirnethylphenyl)-1,3,5-triazin, 2,4-Bis(2-hydroxy-4-propyloxyphenyl)-6-(2,4-dimethylphenyl)-1,3,5-triazin, 2-(2-Hydroxy-4-octyloxyphenyl)-4,6-bis(4-methylphenyl)-1,3,5-triazin, 2-(2-hydroxyl dodecyloxyphenyl)-4,6-bis(2,4-dimethylphenyl)-1,3,5-triazin, 2-(2-Hydroxy-4-tridecyloxyphenyl)-4,6-bis(2,4-dimethylphenyl)-1,3,5-triazin, 2-[2-Hydroxy-4-(2-hydroxy-3-butyloxy-propoxy)-phenyl]-4,6-bis(2,4-dimethyl)-1,3,5-triazin, 2-[2-Hydroxy-4-(2-hydroxy-3-octyloxy-propoxy)phenyl]-4,6-bis(2,4-dimethyl)-1,3,5-triazin, 2-[4-(Dodecyloxy/tridecyloxy-2-hydroxypropoxy)-2-hydroxy-phenyl)-4,6-bis(2,4-dimethylphenyl)-1,3,5-triazin, 2-[2-hydroxy-4-(2-hydroxy-3-dodecyloxy-propoxy)phenyl]-4,6-bis-(2,4-dimethylphenyl)-1,3,5-triazin, 2-(2-Hydroxy-4-hexyloxyphenyl)-4,6-diphenyl-1,3,5-triazin, 2-(2-Hydroxy-4-methoxyphenyl)-4,6-diphenyl-1,3,5-triazin, 2,4,6-Tris[2-hydroxy-4-(3-butoxy-2-hydroxy-propoxy)phenyl]-1,3,5-triazin und 2-(2-Hydroxyphenyl)-4-(4-methoxyphenyl)-6-phenyl-1,3,5-triazin.

Als Costabilisatoren kommen z.B. Eisenverbindungen in Frage, wie sie in der Schrift WO 98/25872, insbesondere auf den Seiten 25 bis 36, beschrieben sind. Sofern diese Verbindungen gefärbt sind, was oftmals der Fall ist, kommt deren Verwendung natürlich nur in Frage, wenn die Eigenfarbe toleriert werden kann.

Weiter ist natürlich von Bedeutung, ob diese Eisenverbindungen für das Anwendungsgebiet das gewünschte Eigenschaftsprofil mit sich bringen. So müssen in der Regel bestimmte Anforderungen an deren Temperaturstabilität im Verarbeitungsprozess und der späteren Verwendung, deren Toxizität bzw. Nichttoxizität, deren Flüchtigkeit usw. erfüllt sein. Die Auswahl möglicher Verbindungen, welche einem gewünschten Eigenschaftsprofil entsprechen, ist jedoch im Normalfall ein Leichtes, da deren pysikalisch-chemische Daten in der Regel bekannt sind oder leicht bestimmt werden können.

Als Polymere, die durch die erfindungsgemäßen und erfindungsgemäß hergestellten Verbindungen stabilisiert werden können, seien beispielsweise genannt:
Polymere von Mono- und Diolefinen, wie z.B. Polyethylen niedriger oder hoher Dichte, lineares Polybuten-1, Polyisopren, Polybutane sowie Copolymerisate von Mono- oder Diolefinen oder Mischungen der genannten Polymeren;
Copolymerisate von Mono- oder Diolefinen mit anderen Vinylmonomeren wie z.B. Ethylen-Alkylacrylat-Copolymere, Ethylen-Alkylmethacrylat-Copolymere. Ethylen-VinylacetatCopolymere oder Ethylen-Acrylsäure-Copolymere;
Polystyrol;
Copolymere von Styrol oder α-Methylstyrol mit Dienen oder Acrylderivaten, wie z.B. Styrol-Butadien, Styrol-Arcylnitril, Styrol-Ethylmethacrylat, Styrol-Butadien-Ethylacrylat, Styrol-Acrylnitril-Methacrylat;
ABS-, MBS- oder ähnliche Polymere;
Halogenhaltige Polymere, wie z.B. Polyvinylchlorid, Polyvinylfluorid, Polyvinylidenflourid sowie deren Copolymer;
Polymere, die sich von α,β-ungesättigten Säuren und deren Derivaten ableiten, wie Polyacrylate, Polymethacrylate, Polyacrylamide und Polyacrylnitrile;
Polymere, die sich von ungesättigten Alkoholen und Aminen bzw. von deren Acrylderivaten oder Acetalen ableiten, z.B. Polyvinylalkohol und Polyvinylacetat;
Polyurethane, Polyamide, Polyharnstoff, Polyester, Polycarbonate, Polysulfone, Polyethersulfone und Polyetherketone.

Weiterhin können mit den erfindungsgemäßen Verbindungen, gegebenenfalls unter Zugabe ein oder mehrerer der vorher genannten Antioxidatien, erfindungsgemäß Lacke stabilisiert werden, vor allem solche Lacke bzw. Lackierungen, welche in hohem Maße der Einwirkung von Umwelteinflüssen, wie Sonnenlicht und Hitze ausgesetzt sind. Dies sind z.B. Lacke für Aussenanstriche, Industrie- oder Fahrzeuglackierungen. Weiter sind dies Lacke für Einbrennlackierungen, vor allem im Automobilsektor.

Die erfindungsgemäßen und erfindungsgemäß hergestellten Verbindungen können in fester oder gelöster Form dem Lack zugesetzt werden. Ihre in der Regel gute Löslichkeit in Lacksystemen ist dabei von besonderem Vorteil.

Bevorzugt werden die erfindungsgemäßen und erfindungsgemäß hergestellten Piperidinderivate der Formel I zum Stabilisieren von Polyolefinen, insbesondere von Ethylen- oder Propylenpolymerisaten, von Polyurethanen sowie von Polyamiden verwendet.

Beansprucht wird im Rahmen der vorliegenden Erfindung zudem stabilisiertes organisches Material, welches erfindungsgemäße oder erfindungsgemäß hergestellte Piperidinderivate der Formel I enthält.

### Beispiel: Synthese des Stabilisators

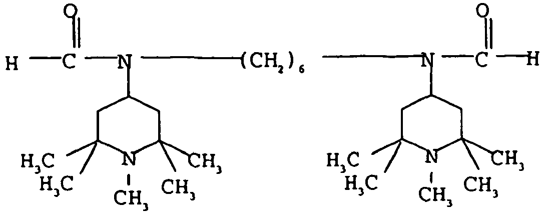

In einem 2-I-Planschliffkolben mit Ankerrührer und Wasserauskreiser werden 337 g des Stabilisators Uvinul^{®} 4050 H (Fa. BASF Aktiengesellschaft, Ludwigshafen; die Formel wurde bereits zuvor gezeigt) in 1200 ml Xylol gelöst. Man setzt 227 g 37%ige Formalinlösung zu, heizt die Mischung auf 80°C auf und tropft innerhalb von 55 Minuten bei 80 - 85°C unter Stickstoffspülung 86 g Ameisensäure zu. Es tritt kräftige Kohlendioxidentwicklung ein. Man erhitzt langsam auf 95°C und rührt die Mischung 2 h nach, wobei gleichzeitig etwa 130 g Wasser ausgekreist werden. Nach Abkühlen auf 80°C Innentemperatur setzt man 600 ml Wasser zu. Anschließend werden bei 140 -160°C Badtemperatur 1,2 I Xylol azeotrop abdestilliert. Das Produkt kristallisiert beim Abkühlen auf Raumtemperatur aus. Es wird von Mutterlauge befreit und im Vakuum getrocknet. Ausbeute: 228 g

### Anwendungstechnische Untersuchung:

Das gemäß vorstehendem Beispiel hergestellte, methylierte Uvinul^{®} 4050 H und sein Ausgangsmaterial Uvinul^{®} 4050 H (Vergleichsstabilisator) wurden jeweils in einer Konzentration von 0,5 Gew.-% mit ABS vom Typ Terluran 877 T (Fa. BASF Aktiengesellschaft, Ludwigshafen) und mit 2 Gew.-% TiO₂ (die Konzentrationsangaben beziehen sich auf die Gesamtmischung) in einem Intensivmischer gemischt und dann über einen Laborextruder bei einer Temperatur von 240°C homogenisiert und granuliert. Das anfallenden Granulat wurde zu 2 mm dicken Prüfkörpem gespritzt und diese in einem Umluftofen bei 90°C gelagert. Gemessen wurde die Vergilbung (Yellowness Index, Yl) nach 0, 250, 500 und 750 h Ofenlagerung.

Der YI-Wert stellt ein Maß für die Effektivität des Stabilisators dar. Je kleiner dieser Wert ist, desto besser ist die Wirkung des Stabilisators.

### YI nach Stunden Ofenlagerung bei 90°C:

| | 0h | 250 h | 500 h | 750 h |
|---|---|---|---|---|
| Uvinul^{®} 4050 H | 10,7 | 11,6 | 13,4 | 20,6 |
| methyliertes Uvinul^{®} 4050 H | 10,4 | 10,6 | 11,2 | 13,7 |

Das erfindungsgemäße, methylierte Uvinul^{®} 4050 H zeigt eine deutlich bessere Stabilisierungsdauer als Uvinul^{®} 4050 H.

## Patentansprüche

1. 4-Formylamino-N-methylpiperidinderivate der allgemeinen Formel (I) 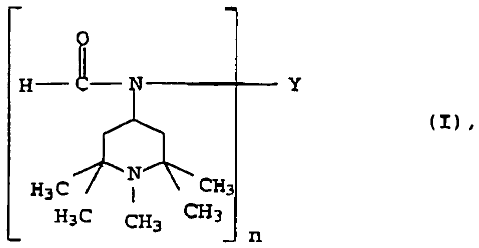 in welcher bedeuten
n einen Wert von 1 oder 2, mit der Maßgabe, dass
für n gleich 1:
Y Wasserstoff oder C₁-C₂₂-Alkyl,
und
für n gleich 2:
Y C₁-C₂₂-Alkylen bedeuten.

2. Verfahren zur Herstellung von Piperidinderivaten der Formel I gemäß Anspruch 1, **dadurch gekennzeichnet, daß** man Verbindungen der Formel I' 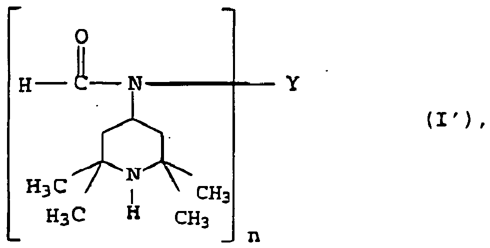 in welcher n und Y die in Anspruch 1 angegebene Bedeutung besitzen, in einem organischen Lösungs-, Emulgier- oder Suspendiermittel, dessen Siedepunkt (bei 1,013 bar) mindestens 70°C beträgt, mit Formaldehyd und anschließend bei einer Temperatur von 70°C bis maximal der Siedetemperatur (bei 1,013 bar) des verwendeten Lösungs-, Emulgier- oder Suspendiermittel mit Ameisensäure umsetzt.

3. Verwendung von Piperidinderivaten der Formel I gemäß Anspruch 1 oder von Piperidinderivaten, hergestellt nach dem Verfahren gemäß Anspruch 2 zum Stabilisieren von organischem Material.

4. Verwendung von Piperidinderivaten der Formel I gemäß Anspruch 1 oder von Piperidinderivaten, hergestellt nach dem Verfahren gemäß Anspruch 2 zum Stabilisieren von Kunststoffen.

5. Verwendung von Piperidinderivaten der Formel I gemäß Anspruch 1 oder von Piperidinderivaten, hergestellt nach dem Verfahren gemäß Anspruch 2 zum Stabilisieren von Lacken.

6. Verwendung von Piperidinderivaten der Formel I gemäß Anspruch 1 oder von Piperidinderivaten, hergestellt nach dem Verfahren gemäß Anspruch 2 als Lichtschutzmittel und Stabilisatoren für Holzoberflächen.

7. Stabilisiertes organisches Material, enthaltend Piperidinderivate der Formel 1 gemäß Anspruch 1 oder Piperidinderivate, hergestellt nach dem Verfahren gemäß Anspruch 2.

## Claims

1. A 4-formylamino-N-methylpiperidine derivative of the general formula (I) 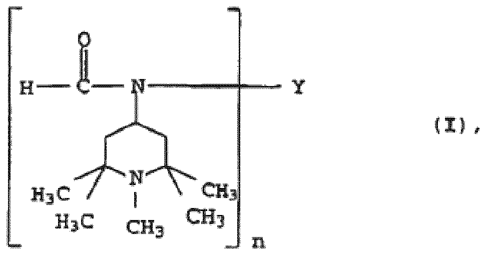 where
n is 1 or 2, with the proviso that
for n = 1:
Y is hydrogen or C₁-C₂₂-alkyl,
and
for n = 2:
Y is C₁-C₂₂-alkylene.

2. The process for preparing piperidine derivatives of the formula I according to claim 1, which comprises reacting compounds of the formula I' 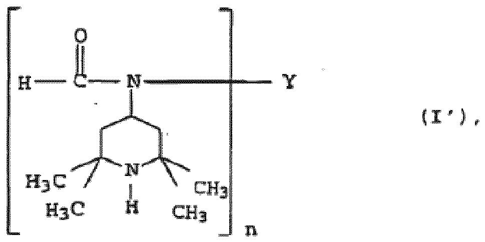 where n and Y are as defined in claim 1, in an organic solvent, emulsification medium or suspension medium whose boiling point (at 1.013 bar) is at least 70°C, with formaldehyde, and then, at from 70°C to not more than the boiling point (at 1.013 bar) of the solvent, emulsification medium or suspension medium used, with formic acid.

3. The use of piperidine derivatives of the formula I according to claim 1, or of piperidine derivatives prepared by the process according to claim 2, for stabilizing organic material.

4. The use of piperidine derivatives of the formula I according to claim 1, or of piperidine derivatives prepared by the process according to claim 2, for stabilizing plastics.

5. The use of piperidine derivatives of the formula I according to claim 1, or of piperidine derivatives prepared by the process according to claim 2, for stabilizing coating materials.

6. The use of piperidine derivatives of the formula I according to claim 1, or of piperidine derivatives prepared by the process according to claim 2, as light stabilizers and stabilizers for wood surfaces.

7. A stabilized organic material comprising piperidine derivatives of the formula I according to claim 1, or piperidine derivatives prepared by the process according to claim 2.

## Revendications

1. Dérivés de 4-formylamino-N-méthylpipéridine de formule générale (I) 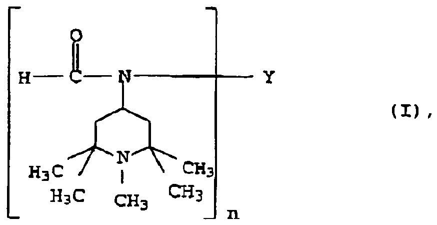 dans laquelle
n signifie une valeur de 1 ou 2, à condition que lorsque n vaut 1 :
Y signifie hydrogène ou C₁-C₂₂-alkyle et
lorsque n vaut 2 :
Y signifie C₁-C₂₂-alkylène.

2. Procédé pour la préparation de dérivés de pipéridine de formule (I) selon la revendication 1, **caractérisé en ce qu'**on transforme des composés de formule I' 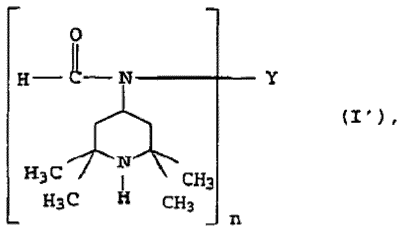 dans laquelle n et Y présentent la signification indiquée dans la revendication 1, dans un solvant, un émulsifiant ou un agent de mise en suspension organique, dont le point d'ébullition (à 1,013 bar) vaut au moins 70°C, avec du formaldéhyde et ensuite à une température de 70°C jusqu'à maximum la température d'ébullition (à 1,013 bar) du solvant, de l'émulsifiant ou de l'agent de mise en suspension avec de l'acide formique.

3. Utilisation de dérivés de pipéridine de formule I selon la revendication 1 ou de dérivés de pipéridine, préparés selon le procédé selon la revendication 2, pour la stabilisation d'une matière organique.

4. Utilisation de dérivés de pipéridine de formule I selon la revendication 1 ou de dérivés de pipéridine, préparés selon le procédé selon la revendication 2, pour la stabilisation de matériaux synthétiques.

5. Utilisation de dérivés de pipéridine de formule I selon la revendication 1 ou de dérivés de pipéridine, préparés selon le procédé selon la revendication 2, pour la stabilisation de laques.

6. Utilisation de dérivés de pipéridine de formule I selon la revendication 1 ou de dérivés de pipéridine, préparés selon le procédé selon la revendication 2, comme agents de protection contre la lumière et comme stabilisants pour surfaces en bois.

7. Matière organique stabilisée, contenant des dérivés de pipéridine de formule I selon la revendication 1 ou de dérivés de pipéridine, préparés selon le procédé selon la revendication 2.
